# EUROPEAN PATENT APPLICATION

(11) **EP 0 548 441 A1**
(43) Date of publication of application: **30.06.1993**
(21) Application number: 91810997.6
(22) Date of filing: 23.12.1991
(51) Int. Cl.: C07K 15/00, A61K 37/24

(54) **Endothelin antagonists**

(71) Applicant: Japat Ltd, CH-4057 Basel (CH)
(72) Inventor: Urade, Yoshihiro, Nishinotoindori, Nakagyo-ku Kyoto (JP); Takai, Michihiro, Toyono-cho, Toyono-gun Osaka (JP); Watakabe, Tadashi, Takarazuka-shi, Hyogo (JP); Yamamura, Takaki, Nishinomiya-shi, Hyogo (JP); Okada,Toshikazu, Takarazuka-shi, Hyogo (JP); Karaki, Hideaki, Bunkyo-ku, Tokyo (JP)
(74) Representative: Schluep, Hans-Peter

(57) **Abstract**

An endothelin antagonistic peptide represented by the formula (I): 
Ac-Xaa1-Xaa2-Xaa3-Ala-Val-Tyr-Xaa4-Ala-His-
    Leu-Xaa5-Ile-Ile-Xaa6    (I)
wherein Xaa₁ represents Asp, Asn or Glu; Xaa₂ represents Lys or Arg; Xaa₃ represents Glu, Gln or Asp; Xaa₄ represents Tyr or Phe; Xaa₅ represents Asp or Gly; and Xaa₆ represents Trp or Phe; as well as a pharmaceutical preparation comprising the peptide.

## Description

### BACKGROUND OF THE INVENTION

### 1. Field of the Invention

The present invention relates to endothelin antagonist, and use thereof.

### 2. Description of Related Art

Endothelin (ET) is family of peptides exhibiting various biological activities including relaxation and contraction of smooth muscle. Nature 332, 4ll, l988, first reported the presence of endothelin-1 (ET-1) and its various biological activities. Pro. Natl. Sci. (USA) 86, 2863, l989, first describes endothelin-3 (ET-3).

In contrast to native ETs having 2l amino acid residue, various ET derivatives have been reported. For example, Eur. J. Pharmacal. 174, 2l-3l, 1989, describes a shortened ET derivative which is a hexapeptide corresponding to a C-terminal portion of native ET as an agonist. Biochem. Biophys. Res. Comm., l63, 424-429, l989, describes ET-l (l-39) derivative and its activities as an agonist.

Eur. J. Pharmacal. 176, l, l990 reports Type II ET receptor present in the bronchus. J. Biol. Chem. 265, 14044, l990, suggests the presence of at least two ET receptors, and the presence of Type II ET receptor in renal mesangial cells. J. Biol. Chem. 265, l7432, l990, describes the presence of Type II ET receptor in the liver.

According to present knowledge involving ETs and ET receptors, ET receptors are classified in at least three different sub-types, i.e., sub-type ET_{A} (or I), ET_{B} (or II) and ET_{C} receptors. Namely, Nature 348, 673, 1990 describes the endothelin receptor subtypes, ET_{A} and ET_{B}. The ET_{A} receptor that may be involved in ET-induced vasoconstriction has a high affinity for ET-l but not for ET-3. The ET_{B} receptor has a high affinity for both ET-l and ET-3.

### SUMMARY OF THE INVENTIONS

ET derivatives that exhibit an antagonistic activity to ET-1 and ET-3 are described in GB Patent Application No. 910072l.l filed on January 12, 1991. The present invention provides novel ET derivatives that exhibit a stronger antagonistic activity to ET-l and ET-3 than the above-cited derivatives.

More specifically, the present invention provides novel ET derivatives represented by the following formula (I) (SEQ ID NO:l): 
Ac-Xaa1-Xaa2-Xaa3-Ala-Val-Tyr-Xaa4-Ala-
His-Leu-Xaa5-Ile-Ile-Xaa6 (I)
wherein Xaaₗ represents Asp, Asn or Glu; Xaa₂ represents Lys or Arg; Xaa₃ represents Glu, Gln or Asp; Xaa₄ represents Tyr or Phe; Xaa₅ represents Asp or Gly; and Xaa₆ represents Trp or Phe.

The present invention also provides a pharmaceutical preparation useful as an ET antagonist comprising the above-mentioned ET derivatives.

### BRIEF EXPLANATION OF THE DRAWINGS

Figure 1 is a graph showing competitive inhibition of 30 pM ^{l25}I-ET-l (with nonlabeled 1 nM ET3) binding to porcine lung membrane by nonlabeled ET-1 (open circle), ET-3 (closed circle) or the present peptide (II) (open triangle).

Fig. 2 is a graph showing competitive inhibition of 10 pM ^{l25}I-ET-3 binding to porcine lung membrane by nonlabeled ET-1 (open circle), ET-3 (closed circle) or the present peptide (II) (open triangle).

Fig. 3 is a graph showing inhibition by the present peptide (II) of smooth muscle contraction (guinia pig trachea) induced by ET-1.

### PREFERRED EMBODIMENTS OF THE INVENTION

The present invention includes any peptides represented by the above-mentioned general formula (I) and having any combination of Xaaₗ , Xaa₂ , Xaa₃ , Xaa₄ , Xaa₅ , and Xaa₆.

The peptide wherein Xaaₗ is Asp, Xaa₂ is Lys, Xaa₃ is Glu, Xaa₄ is Phe, Xaa₅ is Asp, and Xaa₆ is Trp is preferable. This peptide is represented by the formula (II) (SEC ID NO:2):

Ac-Asp-Lys-Glu-Ala-Val-Tyr-Phe-Ala-His-
Leu-Asp-Ile-Ile-Trp (II)

### Production of peptide

The present peptides can be prepared using any conventional procedure for peptide synthesis including a gene recombination technique and chemical synthesis. The peptides are as short as having 14 amino acid residues, and therefore are conveniently prepared by chemical synthesis such as solid phase synthesis.

For example, the present peptides (expressed by the general formula (l) can be prepared by a Conventional procedure using commercial reagents and commercial amino acid synthesizer (for example ABI model 43l). For example the α-amino group of all amino acids is protected by 9-fluoroenylmethyloxy-carbonyl (Fmoc), and functional groups of side chains are protected as follows: a thiol group of cysteine is protected by trityl (Tri), the ε-amino group of lysine is protected by t-butoxy carbonyl (t-Boc), the β-carboxyl group of aspartic acid and γ-carboxy group of glutamic acid are protected by t-butyl ester (OtBu), the phenolic hydroxyl group of tyrosine is protected by t-butyl ether (tBu), and the imidazole group of histidine is protected by trityl (Tri) group.

The synthesis of protected peptides is carried out by solid phase synthesis using an insoluble polystyrene resin derivative (p-benzyloxybenzyl alcohol resin) as a solid carrier. Fmoc group is removed by treatment with 20% piperidine in N-methylpyrolidone (NMP) before condensation reaction at each step. Release from the carrier and deprotection of the synthesized peptide can be carried out by trifluoroacetic acid-water (95/5, v/v) treatment in the presence of l,2-ethanedithiol.

### Biological activities

The present peptides exhibit remarkable antagonistic activities to ET. The activities were demonstrated by inhibition of the binding of ET-1 and ET-3 to porcine lung ET receptors and inhibition of the contraction of guinea-pig trachea.

### (1) Inhibition of the binding of [¹²⁵I] ET-1 and [¹²⁵I] ET-3 to ET receptors in porcine lung membranes

### Binding Assay

Porcine lung was homogenized with a Kinematica Polytron homogenizer (Lucern, Switzerland) three times at top speed for each 30 sec in 9 volumes of ice-cold 0.25 M sucrose solution containing 20 mM Tris-HCl (pH 7.4), 0.2 mM phenylmethylsulfonyl fluoride, 1 µM leupeptin, 1 µM pepstatin, 0.1 mM EDTA, and 0.5 mM EGTA. After centrifugation of the homogenate at 1,000 x g for 10 min at 4°C, the supernatant was centrifuged again at 20,000 x g for 20 min. The resulting pellet was washed three times as described above and used as a membrane source. The membrane was stocked in aliquots at -80°C until use.

The plasma membrane of porcine lung (∼2 µg protein) was incubated at 37°C for l h with 30 pM [^{l25}I] endothelin (ET)-l or 10 pM [¹²⁵I] ET-3 in the absence and presence of various amounts of nonlabeled ligands in a total volume of 1 ml of 20 mM HEPES (pH 7), 145 mM NaCl, 5 mM KCl, 3 mM MgCl₂ ,
1 mM EGTA, 1 mg/ml bovine serum albumin, and 0.2 mg/ml bacitracin. After the incubation, unbound [^{l25}I]ET was separated by centrifugation at 20,000 x g for 20 min at 4°C followed by aspiration of the supernatant. The radioactivity in membrane pellet was measured in a gamma counter. Nonspecific binding was defined as membrane-associated radioactivity in the presence of saturating concentrations of ET(l00 nM). Nonspecific binding was subtracted from the total binding and the difference was defined as specific binding. Total binding was always less than l5% of the total radioactivity added.

### Characterization of ET Binding Activity in Membranes of Porcine Lung

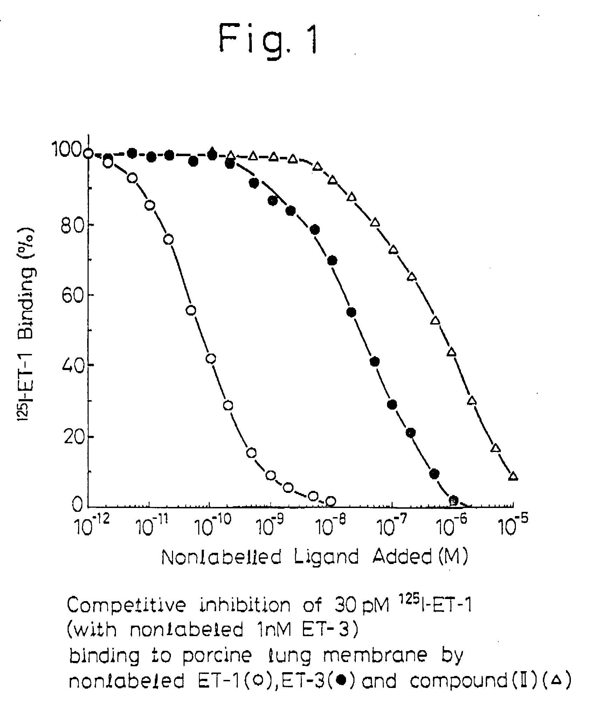

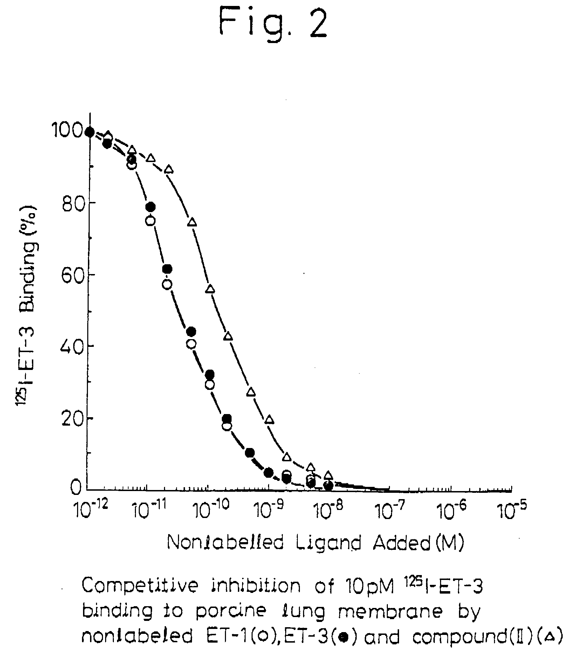

The specific binding site for ETs in the plasma membrane of porcine lung was detected. The binding to the ET_{A} receptor was determined with [¹²⁵I]ET-1 in the presence of 1 nM unlabeled ET-3 and the binding to the ET_{B} receptor was determined with [¹²⁵I]ET-3 alone. By Scatchard analyses, the ET_{A} receptor showed an apparent dissociation constant (Kd) of 44 pM and maximum binding sites (Bmax) of 342 fmol/mg protein, while the ET_{B} receptor had a Kd of 8 pM and Bmax of 362 fmol/mg protein (Table 1). The binding assays were performed as described above. From the inhibition curves for the binding of [^{l25}I] ETs, the apparent binding affinity constant (Ki) of each analog was calculated as a parameter of the affinity to the ET_{A} and ET_{B} receptors. Results are shown in Figs. 1 and 2, as well as Table 1.

**Table 1**

| Characterization of Endothelin Binding Activity in Membranes of Porcine Lung and Ki Value of Compound (II) Against[^{l25}I]-ET-l or[¹²⁵I]-ET-3 Binding | | |
|---|---|---|
| | [^{l25}I] ET-l | [¹²⁵I] ET-3 |
| K_{D} | 44 pM | 8 pM |
| Bmax | 342fmol/mg protein | 362fmol/mg protein |
| ET-l (Ki) | 40 pM | 8 pM |
| ET-3 (Ki) | 15 nM | 8 pM |
| Compound (II) (Ki) | 450 nM | l20 pM |

### (2) Inhibition of the Contraction of Guinea Pig Trachea

The contraction of the guinea pig trachea was measured according to Br. J. Pharmacol. 98, 483, l989, by the following procedure.

Male guinea pigs weighing 350 - 500 g were stunned and bled. The trachea was rapidly removed and placed in an oxygenated (95% O₂ , 5% CO₂) Krebs solution. After removal of the adherent fat and connective tissues, the trachea was cut into 2 mm wide rings. The two rings were tied together, end to end, placed in a 30 ml organ bath and connected to an isometric force-transducer under an initial tension of 1.0 g. After equilibration for 60 min, the preparations were exposed to ET-l, -3 and the synthetic peptide analogs. A high KC1 (60 mM) solution was used as a reference standard resulted in the maximum contraction.

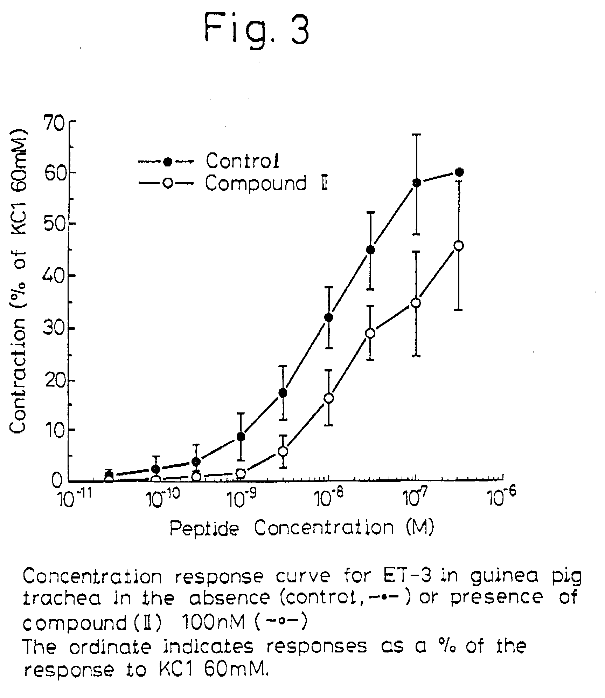

As shown in Fig. 3, ET-3 caused a contraction of the trachea in a concentration dependent manner. The contraction was significantly inhibited by l00 nM compound II which was added 5 min prior to the addition of ET-3.

Since the present peptides are antagonistic to ET-1 and ET-3, they are promising for medication of any symptoms that are medicated through ET receptors in particular an ET receptor subtype to which both ET-l and ET-3 have a high affinity. For example, the present peptides are promising as an anti-asthma drug, a drug for renal failure, a drug for glaucoma, an antiinflammation drug, and a drug for heart failure, and also, in general, useful for medication of any symptoms which are mediated through endothelin receptors in particular an endothelin receptor subtype ET_{B}. Moreover, the present peptides are good reagents for clarifying physiological functions of endothelin family peptides.

Therefore, the present invention provides a pharmaceutical preparation comprising a peptide represented by the formula (I), and a pharmaceutically acceptable carrier.

Namely, the peptide of the present invention can be used, for example, in the manufacture of pharmaceutical preparations that contain a therapeutically effective amount of the derivative together or in admixture with inorganic or organic, solid or liquid, pharmaceutically acceptable carriers that are suitable for parenteral, for example, intramuscular, intravenous, nasal, subcutaneous or intraperitoneal, administration.

For parenteral administration suitable injection or infusion solutions, preferably isotonic aqueous solutions or suspensions, are available, it being possible to prepare these before use from, for example, lyophilized preparations that contain the active ingredient alone or together with a pharmaceutically acceptable carrier, for example, mannitol. Such preparations may be sterilized and/or contain adjuncts, for example preservatives, stabilizers, wetting agents and/or emulsifiers, solubilizers, salts for regulating the osmotic pressure, and/or buffers.

The present pharmaceutical preparations may contain, if desired, further pharmacologically valuable substances and are manufactured in a manner known per se, for example by conventional mixing, dissolving or lyophilizing processes. They contain approximately 0.1% to l00%, especially approximately 1% to approximately 50%, or, in the case of lyophilizates, up to l00% of the active ingredient.

Depending upon the nature of the disease and the condition of the organism, parenteral doses of from approximately l µg to approximately l0 mg are given for the treatment of warm-blooded animals (humans and animals) weighing approximately 70 kg.

### Examples

The present invention will now be further illustrated by, but by no means limited to, the following examples.

### Example l. Synthesis of Ac-endothelin-1 (8-21) (Compound II)

463 mg (0.25 mmol) of Fmoc-Trp-OCH₂-phenyloxybenzyl resin (a product of Nova biochem) was treated with 4.5 ml 20% piperidine in NMP for l8 min. After the piperidine treatment, the peptide resin was washed with NMP.

353 mg (1 mmol) of Fmoc-Ile was dissolved in 2.l ml of NMP, to this mixture were added l.6 ml of l M 1-hydroxy benzotriazol (HOBt) and l.3 ml of l M dicyclohexyl carbodiimide (DCCD). The mixutre was stirred for 24 min. and then added to amino acid (peptide) resin. After reaction (7l min) the peptide resin was washed with NMP in preparation for the next step. One step was accomplished in about ll0 min.

The above step was repeated using l mmol each of the following protected amino acids: Fmoc-Ile, Fmoc-Asp(OtBu), Fmoc-Leu, Fmoc-His(Tri), Fmoc-Ala, Fmoc-Phe, Fmoc-Tyr(tBu), Fmoc-Val, Fmoc-Ala, Fmoc-Glu(OtBu), Fmoc-Lys(Boc) and Fmoc-Asp(OtBu) in this order, to obtain a protected peptide resin. Fmoc-Asp(OtBu)-Lys(Boc)-Glu(OtBu)-Ala-Val-Tyr(tBu)-Phe-Ala-His(Tri)-Leu-Asp(OtBu)-Ile-Ile-Trp-OCH₂-phenyloxybenzyl-resin.

The Protected peptide resine was treated with 20% piperidine in NMP for l8 min. to remove Fmoc group. 47 µl (0.5 mmol) of acetic anhydride in 5 ml of dimethylformamide (DMF) and 70 µl (0.5 mmol) of triethylamine were added to the resin. The mixture was stirred at room temperature for 1 hour. The Protected Peptide resin was collected by filtration and washed with DMF and methylene chloride. 1 ml of 1,2-ethanedithiol and 4 ml of 95% TFA were added to the protected peptide resin, and the mixture was stirred at room temperature for 5 min. The resin and solution are then separated by filtration, and the resin was washed with trifluoroethanol and methylene chloride. The filtrate was evaporated under reduced pressure and then isopropylether-n-hexane (l/1, v/v) was added. The resulting powder was collected and washed with the same solvent and then dried over.

The powder was retreated with 1 ml of 1,2-ethanedithiol and 4 ml of 95% TFA at room temperature for 90 min., and then evaporated. The thus obtained residue was then precipitated by adding Isopropylether-n-hexane (1/1, v/v). The desired product was purified by preparative high performance liquid chromatography (HPLC) to obtain 159 mg of a peptide represented by the following formula: 
Ac-Asp-Lys-Glu-Ala-Val-Tyr-Phe-Ala-His-Leu-
Asp-Ile-Ile-Trp (Compound II)
The thus obtained peptide provided a single peak in analytical HPLC on Toso TSK-Gel ODS 120-T at a duration time of 23.8 min.

Result of amino acid analysis by acid hydrolysis: Asp 2.17(2), Glu 1.03(1), Ala 2.00(2), Val 0.99(1), Ile 1.21(2), Leu 1.03(1), Tyr 1.0l(l), Phe 1.04(1), His 1.03(1), Lys 1.01(1), Trp 0.94(l)

SEQUENCE LISTING
SEQ ID NO:1
SEQUENCE TYPE: Peptide
SEQUENCE LENGTH: 14
TOPOLOGY: linear
MOLECULE TYPE: synthetic peptide
FEATURES:
The first Xaa = Asp, Asn or Glu
The second Xaa = Lys or Arg
The third Xaa = Glu, Gln or Asp
The seventh Xaa = Tyr or Phe
The 11th Xaa = Asp or Gly
The 14th Xaa = Trp or Phe

Xaa Xaa Xaa Ala Val Tyr Xaa Ala His Leu Xaa Ile Ile Xaa 14
1               5                   10

SEQ ID NO:2
SEQUENCE TYPE: peptide
SEQUENCE LENGTH: 14
TOPOLOGY: linear
MOLECULE TYPE: synthetic peptide
SEQUENCE:

Asp Lys Glu Ala Val Tyr Phe Ala His Leu Asp Ile Ile Trp 14
1               5                   10

## Claims

1. A peptide represented by the formula (I) [SEQ NO:l]: 
Ac-Xaa1-Xaa2-Xaa3-Ala-Val-Tyr-Xaa4-Ala-
His-Leu-Xaa5-Ile-Ile-Xaa6 (I)
 wherein Xaa₁ represents Asp, Asn or Glu; Xaa₂ represents Lys or Arg; Xaa₃ represents Glu, Gln or Asp; Xaa₄ represents Tyr or Phe; Xaa₅ represents Asp or Gly; and Xaa₆ represents Trp or Phe.

2. A peptide according to claim 1 wherein the peptide has the following formula (II) [SEQ NO:2]:

Ac-Asp-Lys-Glu-Ala-Val-Tyr-Phe-Ala-His-
Leu-Asp-Ile-Ile-Trp (II)

3. A pharmaceutical preparation comprising a peptide of the formula (I) defined in claim l and a pharmaceutically acceptable carrier.
